# EUROPEAN PATENT SPECIFICATION

(11) **EP 4 225 509 B1**
(45) Date of publication and mention of the grant of the patent: **26.06.2024**
(21) Application number: 21785926.3
(22) Date of filing: 12.10.2021
(51) Int. Cl.: B06B 1/06, B05B 17/00, B05B 17/06, A61M 11/00, A61M 15/00

(54) **AEROSOL GENERATOR FOR AN INHALATION DEVICE**
AEROSOLGENERATOR FÜR EINE INHALATIONSVORRICHTUNG
GÉNÉRATEUR D'AÉROSOL POUR DISPOSITIF D'INHALATION

(30) Priority: 12.10.2020 EP 20201389
(43) Date of publication of application: 16.08.2023
(73) Proprietor: Vectura Delivery Devices Limited, Chippenham, Wiltshire SN14 6FH (GB)
(72) Inventor: HUBER, Martin, Cambridge Cambridgeshire CB4 0GZ (GB); SCHWENDNER, Sebastian, Cambridge Cambridgeshire CB4 0GZ (GB); CEREDA, Lelio, Cambridge Cambridgeshire CB4 0GZ (GB); NOTT, Werner, 9330 Althofen (AT)
(74) Representative: Clarke, Christopher John
(86) International application number: PCT/EP2021/078198
(87) International publication number: WO 2022/079037

(56) References cited:
- US-A1- 2003 047 620
- US-A1- 2017 059 378
- US-A1- 2019 329 281
- US-A1- 2020 306 797

## Description

### Technical Field of the Invention

The present invention relates to an aerosol generator for an inhalation device, in particular a vibrating mesh nebulizer.

### Background

Aerosols for medical inhalation therapy generally comprise an active ingredient dissolved or suspended in an aerosolisable liquid (often water). A homogeneous distribution of aerosol droplets with a droplet size of around 5 µm is required in order to reach deep into the lungs. Vibrating mesh nebulizers are one type of device for producing such aerosols. These devices comprise a vibrator, such as piezoelectric element which is excited at ultrasonic frequencies in order to induce vibration; a membrane (sometimes called a mesh), which has a large number of micro-pores (i.e. through holes) which typically have a diameter of 1 µm to 10 µm; and a reservoir, which supplies the liquid drug formulation to the membrane. Such nebulizers typically have a piezoelectric element ("piezo") in the form of an annular ring, with one electrical contact (e.g. positive) on its upper surface and the other electrical contact (e.g. negative) on its lower surface.

Many vibrating mesh nebulizers have an annular piezo with the membrane arranged over the central hole. The membrane is either directly attached to the piezo, or the mesh and the piezo are both attached to a supporting substrate, such as a planar metal ring. The piezo expands and contracts radially in response to an applied voltage, thereby flexing the membrane, directly, or via the substrate. Such nebulizers are disclosed, for example, in US 2003/047620, US 9027548, WO 2012/046220 and WO 2015/193432. US 2010/0044460 discloses a vibrating mesh nebulizer that operates in a different manner. The piezo is attached to a flange located towards one end of a transducer, and the membrane is attached to the other end. The piezo causes the transducer to vibrate longitudinally, which in turn passes the vibrations on to the membrane. Thus the membrane vibrates in a longitudinal "piston" mode, instead of being flexed by radial vibration of the piezo. In each type of vibrating mesh nebulizer, a voltage is applied across the piezo by means of two electrical contacts, one on each side. For example, a metal substrate may form the contact on one side, and a pin may contact a conductive layer applied to the other side. Each contact has a wire or other connector, such as a flexible strip connector, for connection to the source of electrical power. This type of arrangement necessitates a number of different components. US 2019/329281 discloses a nebulizer of the first type, in which the two electrical contacts to the piezo are located on the on the same surface.

### Brief Description of the Invention

The present inventors have identified an improved way of arranging the electrical contacts to the piezo in an aerosol generator. In a first aspect, the present invention provides an aerosol generator comprising a vibratable membrane, a support member, an annular piezoelectric element having a first surface with a first conductive region, a second surface with a second conductive region, an inner edge and an outer edge. The second conductive region extends across at least part of the inner edge or the outer edge onto the first surface of the piezoelectric element to form a contact region. The first conductive region and the contact region are spaced apart on the first surface. The aerosol generator further comprises a flexible connector having a surface which is an electrical insulator with first and second conductive regions that correspond to the first conductive region and to the contact region on the piezoelectric element respectively. The flexible connector has two 'S' shaped legs for making electrical connection to a controller that provides a driving current to the piezoelectric element.

The term "S-shaped" means that the legs have two bends, curves or corners which are in opposite senses. The bends/curves/corners may be such that the legs lie in the plane of the flexible connector. Alternatively, the bends/curves/corners may be such that the legs are arranged out of the plane of the flexible connector.

The second conductive region on the piezoelectric element may extend across part of the outer edge or across part of the inner edge to form the contact region on the first surface. The second conductive region on the piezoelectric element may extend across the whole of the outer edge or the whole of the inner edge to form the contact region.

The first and second conductive regions may cover most of the first and the second surfaces of the piezoelectric element respectively.

The piezoelectric element may be connected to the flexible connector by a layer of anisotropic conducting paste or by anisotropic conductive adhesive transfer tape.

The conductive regions on the piezoelectric element may be stencilled silver layers.

The support member of the aerosol generator may comprise a hollow tubular body having a flange at, or close to, a first end, onto which the piezoelectric element is attached, and a second end into or onto which the membrane is mounted. Alternatively, the support member may comprise an essentially planar annulus or disk, and the membrane may be in contact with the piezoelectric element, or the membrane and the piezoelectric element may be mounted on the support member, for example on opposite sides of the support member.

In a second aspect, the present invention provides an inhalation device comprising the aerosol generator of the first aspect of the invention. The inhalation device may comprise an aerosol head comprising the aerosol generator; a base unit having one or more an air inlet openings, an air outlet opening, and a groove; and a mouthpiece component which is insertable into the groove and which has an air inlet opening that is attachable to the air outlet opening of the base unit, a lateral opening for receiving the aerosol generator, and an aerosol outlet opening; wherein the base unit, the mouthpiece component and the aerosol head are detachably connectible with each other.

### Brief Description of the Figures

Figure 1 shows an expanded view of a known aerosol generator.
Figures 2A and 2B show the piezo used in the aerosol generator of Figure 1.
Figure 3 shows an expanded view of an aerosol generator according to the invention.
Figures 4A and 4B show the electrical contacts on a piezo for use in the aerosol generator of Figure 3.
Figure 5 shows a flexible connector for use with the piezo of Figure 4.
Figure 6 shows a cross-section through the interface between the piezo and the flexible connector in the aerosol generator of Figure 3.
Figures 7A and 7B show a further flexible connector for use with the piezo of Figure 4.
Figures 8A and 8B show a second configuration of the electrical contacts on a piezo.
Figure 9 shows a flexible connector used with the piezo of Figure 8.
Figures 10A and 10B show a third configuration of the electrical contacts on a piezo.
Figures 11A and 11B show a fourth configuration of the electrical contacts on a piezo.
Figure 12 shows an expanded view of a vibrating membrane nebulizer device which uses an aerosol generator according to the invention.

### Detailed Description of the Invention

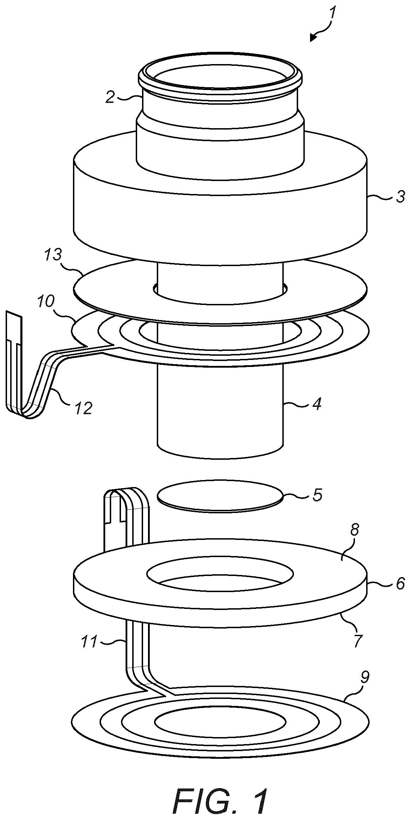

Figure 1 shows an expanded view of a known aerosol generator of the type disclosed in US 2010/0044460. The aerosol generator **1** has a transducer **2** formed from a hollow tubular stainless steel body **4** with a flange **3** having a larger wall thickness which acts as a stress concentration zone towards one end. The membrane **5,** which has a large number of holes with openings in the range from about 1 µm to about 10 µm, is mounted on or just inside the other end of the tubular body. The internal volume of the tubular body forms a reservoir into which the liquid to be nebulized is filled.

The transducer **2** is designed so that small vibrations of the piezo **6** are amplified into larger vibrations of the membrane **5.** The piezo **6** is an annular single or multilayer ceramic and is thicker than the piezos typically used in aerosol generators in which the membrane is directly in contact with the piezo (or only spaced apart by an essentially planar substrate). The stress concentration zone **3** has a relatively large mass. When the piezo **6** is actuated, it vibrates longitudinally, i.e. in a direction parallel to the symmetry axis of the transducer **2,** causing micronic displacements of the flange. These are amplified by the tubular body **4** of the transducer and cause the membrane **5** to vibrate in a longitudinal mode, typically at a frequency in the range of 50 to 200 kHz range. Vibration of the membrane leads to the formation and emission of aerosol droplets through the holes. The membrane may be made of plastic, silicon, ceramic or more preferably metal, and may be affixed at or near to the end of the transducer by various means, such as gluing, brazing, crimping or laser welding.

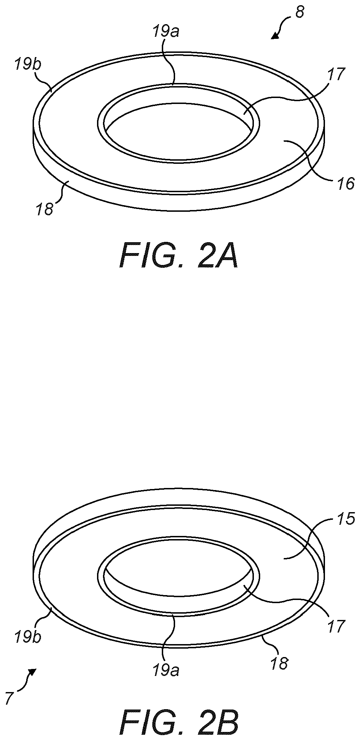

Figures 2A and 2B show the upper **8** lower **7** and surfaces of the piezo **6** respectively. A conductive silver stencil layer **15** covers the lower surface **7,** apart from uncoated regions **19a** at the inner edge **17** and **19b** at the outer edge **18.** Similarly, a second conductive silver stencil layer **16** covers the upper surface **8,** apart from the uncoated regions **19a, 19b.** The silver stencil layers **15, 16** form the two electrical contacts, and the uncoated regions **19a, 19b** prevent a short circuit between the contacts.

First **9** and second **10** flexible electrical connectors abut the lower **7** and upper surfaces **8** of the piezo respectively. The connectors each have a leg **11, 12,** through which electrical connection is made to a printed circuit board (PCB). The connectors are bonded to the piezo with a conductive adhesive, for example anisotropic conductive film (ACF); the second connector **10** (and hence the piezo) is also bonded to the lower side of the flange **3,** for example by epoxy glue **13.** The connectors form an electrical connection to the silver layers through the conductive adhesive, so that an electric field can be applied across the piezo.

In the configuration shown in Figure 1, the second flexible connector **10** is located between the piezo **6** and the flange **3.** Thus the second flexible connector **10** could absorb some of the mechanical energy from the piezo, and hence damp the vibrations. This can be avoided by an alternative configuration in which the second flexible connector **10** is located on the other side of the flange **3** , so that the piezo **6** is attached directly to the flange. In this alternative configuration, the electrical connection from the second flexible connector **10** to the upper side of the piezo **8** is made though the flange **3** (which is metallic). However, it is necessary to form a good electrical and mechanical connection between the flange **3** and the upper surface **8** of the piezo **6,** which can be difficult to achieve.

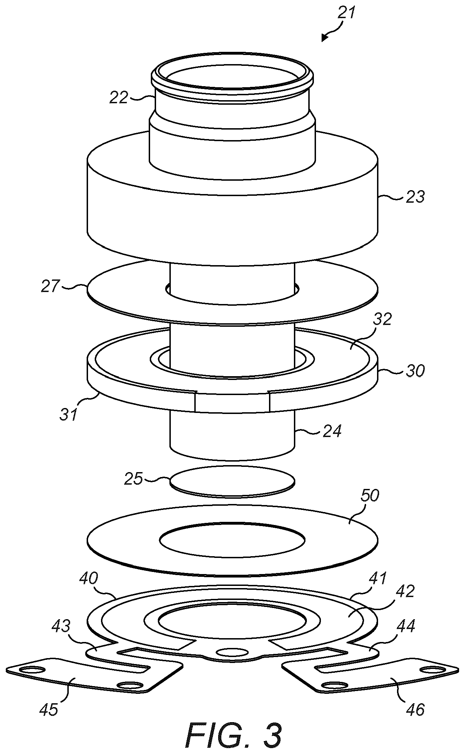

Figure 3 shows an expanded view of an aerosol generator **21** according to the invention, which is similar to that of Figure 1. The transducer **22** has a flange **23** to which the piezo **30** is attached, for example by epoxy glue **27,** and a tubular body **24** with a membrane **25** at its end, as in Figure 1. However, in Figure 3, there is only one flexible connector **40,** which abuts the lower surface **31** of the piezo **30.** The upper surface **32** of the piezo **30** is bonded directly to the lower side of the flange **23.** The flexible connector **40** has an annular contact part **41** with an upper surface **42** and two legs **43, 44** through which electrical connection is made to a PCB at the feet **45, 46.** It is bonded to the piezo by a layer of anisotropic conducting paste **50** (ACP).

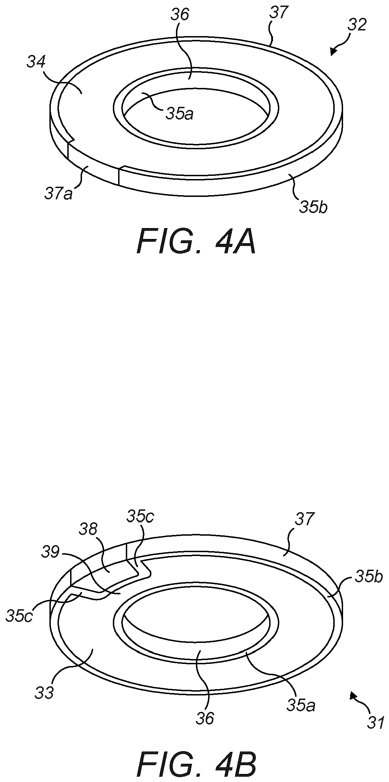

Figures 4A and 4B show the upper **32** and lower **31** surfaces of the piezo respectively. First and second conductive silver stencil layers **33, 34** cover most of the lower (first) surface **31** and the upper (second) **32** surface respectively. There is an uncoated region **35a** at the inner edge **36** of the piezo, as in Figures 2A and 2B. Another uncoated region **35b** occupies most of the outer edge **37.** However, in contrast to Figures 2A and 2B, there is a detour **35c** in the uncoated region **35b** away from the outer edge **37** on the lower surface **31,** so that the first conductive layer **33** has a narrow part **39.** The second conductive layer **34** correspondingly extends across part of the outer edge **37a** and onto the lower surface to form a small contact region **38** defined by the detour **35c.** The uncoated detour **35c** separates the contact region **38** from the first conductive layer **33** so that current cannot flow directly between the first and second conductive layers.

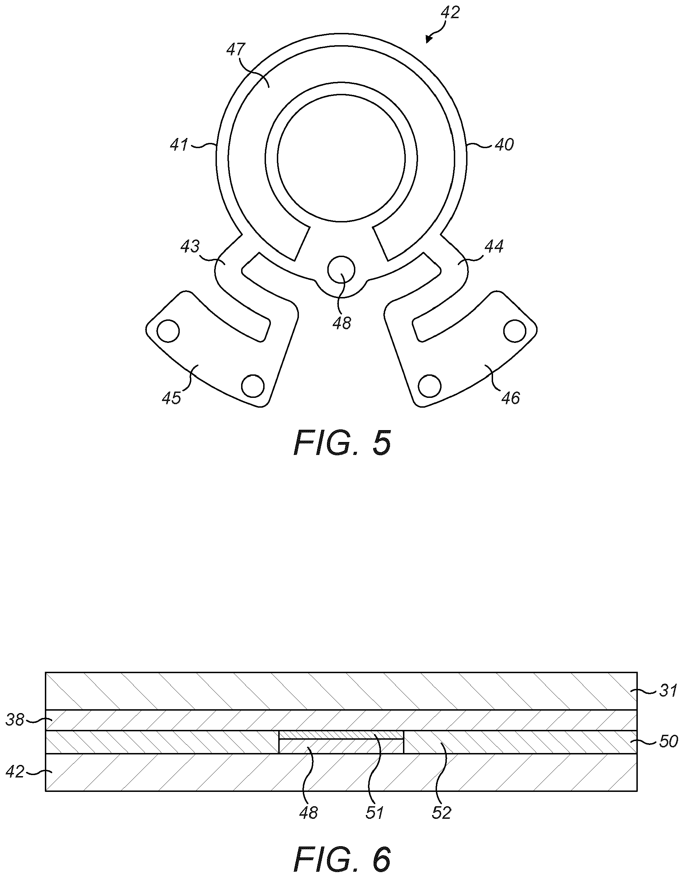

Figure 5 shows the upper surface **42** of the flexible connector **40,** which, when assembled in the aerosol generator, faces the lower surface of the piezo. The surface layer of the connector **40** is an electrical insulator (such as polyimide), apart from two conductive regions **47, 48** formed for example from gold-coated copper. These regions respectively correspond to the locations of the first conductive layer **33** and the small contact region **38** of the second conductive layer on the piezo. The conductive regions **47, 48** connect to a PCB via tracks inside each leg **43, 44** which terminate in a contact point on each foot **45, 46.** The legs lie in the same plane as the annular contact part **41** and are S-shaped (when viewed from above), which makes them more flexible. This decouples the piezo from the fixed connections between the feet **45, 46** of the flexible connector and the PCB. This minimizes damping of the transducer vibrations by the flexible connector, which would otherwise reduce the aerosol output rate from the membrane.

Figure 6 shows a cross-section through the interface between the small contact region **38** on the lower surface **31** of the piezo and the conductive region **48** on the upper surface **42** of the flexible connector, between which is a layer of anisotropic conducting paste **50.** The ACP contains conductive particles in a non-conductive binder. When heat and pressure is applied, a thin layer **51** of ACP is formed between the contact region **38** and the conductive region **48;** a thicker layer **52** is formed where there is no conductive region on the flexible connector. The thin layer **51** is sufficiently thin that the conductive particles in the ACP span the gap between the contact region **38** and the conductive region **48,** and hence form an electrical connection. (A thin layer is similarly formed between the other conductive regions **33, 47**). However, the thicker layer **52** is wider than the size of the conductive particles, so the particles remain isolated from each other within the non-conductive binder. Thus there is no electrical connection through the thicker layer **52,** which prevents short circuits. The flexible connector could alternatively be attached to the piezo in other ways that prevent short circuits, for example by using a non-conductive glue and appropriate masks.

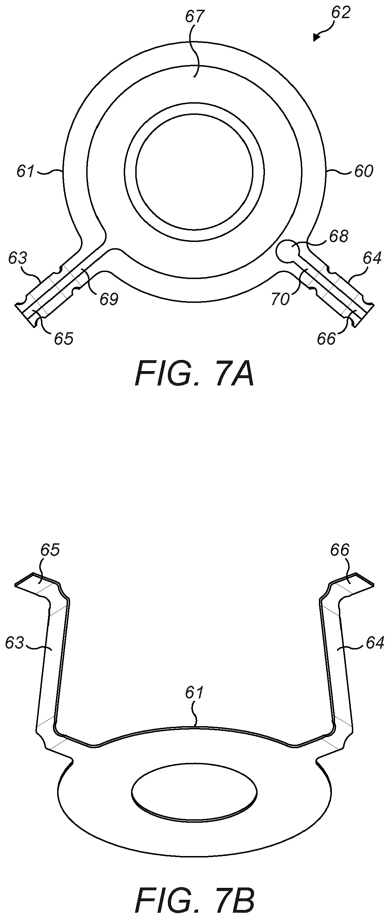

Figures 7A & 7B show a second embodiment of a flexible connector **60** for use with the piezo of Figures 4A and 4B. Figure 7A shows the upper surface **62** of the flexible connector **60,** which, when assembled in the aerosol generator, faces the lower surface of the piezo. The surface layer of the connector **60** is an electrical insulator (such as polyimide), apart from two conductive regions **67, 68** formed, for example, from gold-coated copper. These regions respectively correspond to the locations of the first conductive layer **33** and the small contact region **38** of the second conductive layer on the piezo, but are arranged in a different way from the flexible connector of Figure 5. The first conductive region **67** is in the form of a complete ring situated towards the inner edge of the annular contact part **61,** so that it is in contact with the first conductive layer **33,** and does not come into contact with the small contact region **38.** It connects to the first track **69** which runs along the first leg **63.** The second conductive region **68** is in the form of a small circle (similar to the second conductive region **48** in Figure 5), and connects to the second track **70** which runs along the second leg **64.** The tracks **69, 70** on each leg **63, 64** terminate in a contact point on each foot **65, 66,** which is connected to the PCB as before. Figure 7B shows the flexible connector in a perspective view from below (so that its upper surface is not visible). The legs **63, 64** are bent out of the plane of the annular contact part **61,** and are S-shaped when viewed from the side. Thus the S shape lies in a different plane compared to the flexible connector of Figure 5, but nonetheless increases the flexibility of the legs in a similar manner. This decouples the piezo from the fixed connections between the feet **65, 66** of the flexible connector and the PCB, which minimizes damping of the transducer vibrations by the flexible connector.

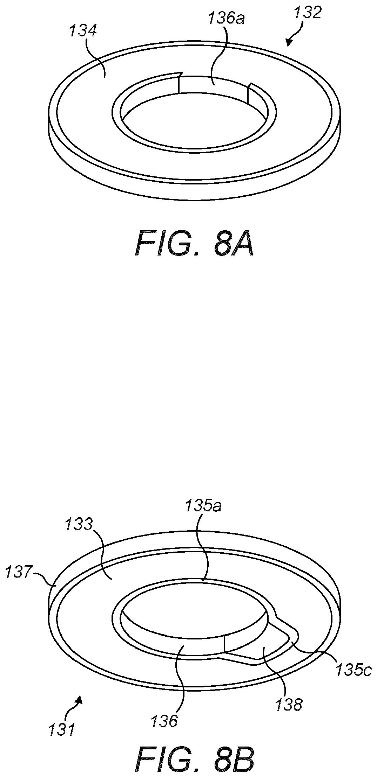

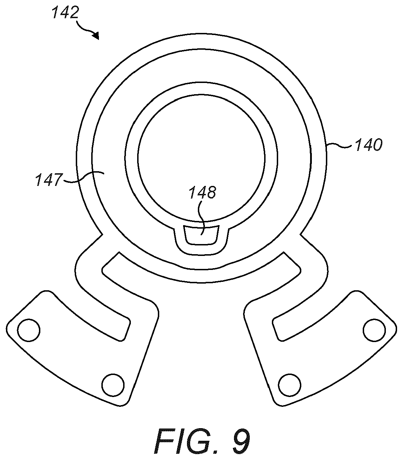

Figures 8A and 8B show an alternative configuration of the conductive silver stencil layers **133,134** on the piezo. This is similar to the embodiment of Figures 4A and 4B, except that the detour **135c** is formed in the uncoated region **135a** at the inner edge **136** (rather than the outer edge **137**). The second conductive layer **134** covers most of the upper surface **132** and also extends across part of the inner edge **136a** and onto the lower surface to form a small contact region **138.** As shown in Figure 9, the two conductive regions **147, 148** on the upper surface **142** of the flexible connector **140** are shaped to correspond to the first conductive layer **133** and the small contact region **138** on the piezo.

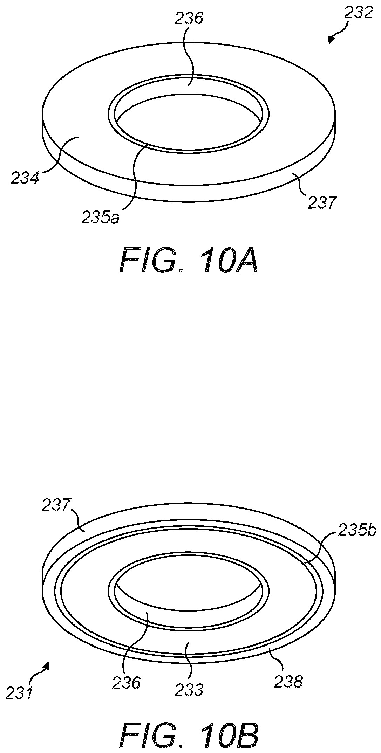

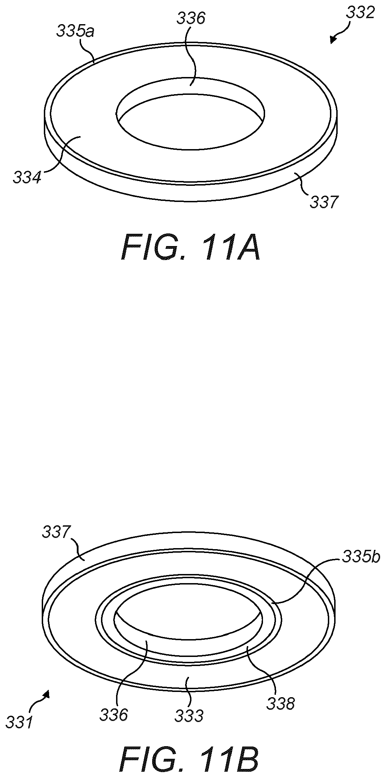

Figures 10A and 10B show a variant of the embodiment of Figures 4A and 4B in which there is an uncoated region **235a** at the inner edge **236** of the piezo as before, but the uncoated region **235b** is located at a short distance onto the lower surface **231** around the whole of the outer edge **237.** The second conductive layer **234** extends over the whole of the outer edge **237** and onto the lower surface **231** to form an annular contact region **238.** Figures 11A and 11B show a variant of the embodiment of Figures 8A and 8B in which there is an uncoated region **335a** at the outer edge **337** of the piezo as before, but the uncoated region **335b** is located at a short distance onto the lower surface **331** around the whole of the inner edge **336.** The second conductive layer **334** extends over the whole of the inner edge **336** and onto the lower surface **331** to form an annular contact region **338.** In each case, the two conductive regions on the upper surface of the flexible connector (not shown) are shaped to correspond to the conductive layers **233, 238** and **333, 338** on the piezo respectively.

Having both contacts on this same side of the piezo has the advantage that a single connector with both the positive and negative connections can be used, instead of two connectors as in known aerosol generators. Thus fewer components are required, which reduces the cost and simplifies the assembly process. Having fewer components also improves the reliability and lifetime of the aerosol generator because it removes potential points of failure.

It would be possible to simply have two connections on one side of the piezo with no conductive region on the other side. However, this would result in reduced membrane vibration and hence poor performance, because the electrical field applied between the contacts would not properly activate all of the piezo. In the present invention, the conductive layers cover almost all of the surfaces, so the electrical field is applied uniformly across the whole piezo. This results in uniform deformation of the piezo, and hence good membrane vibration, whilst still reaping the benefits of having fewer components. Also, maximizing the area of the contact on the piezo minimizes the resistance arising from the contact.

While the configurations of contacts shown in Figures 4A & 4B, 8A & 8B, 10A & 10B and 11A & 11B all work well, the configuration of Figures 4A & 4B is preferred. This is because it is simpler to produce a conductive layer that wraps over a small region of the outer edge of the piezo than either a layer that wraps over part or all of the inner edge (as in Figures 8A & 8B and 11A & 11B), or around the whole, or a large part of, the outer edge (as in Figures 10A & 10B).

While the invention has been described with reference to an aerosol generator of the type described in US 2010/0044460, in which the membrane is spaced apart from the piezo by a tubular transducer body, it can also be used in aerosol generators of the types described in US 2003/047620, US 9027548, WO 2012/046220 and WO 2015/193432, in which the membrane is directly in contact with the piezo, or only spaced apart by an essentially planar support member.

Nonetheless, the invention is especially advantageous in aerosol generators of the type described in US 2010/0044460, because damping arising from the connectors is a particular concern in these. Since the transducer amplifies small vibrations of the piezo into larger vibrations of the membrane, any damping of the small vibrations would also be amplified. This would result in reduced membrane vibration, and hence a reduced aerosol output rate. Replacing two flexible connectors with a single flexible connector avoids the need to either interpose a flexible connector between the piezo and the flange (which causes damping) or to form an electrical, as well as mechanical connection between the piezo and the flange (which can be difficult to achieve).

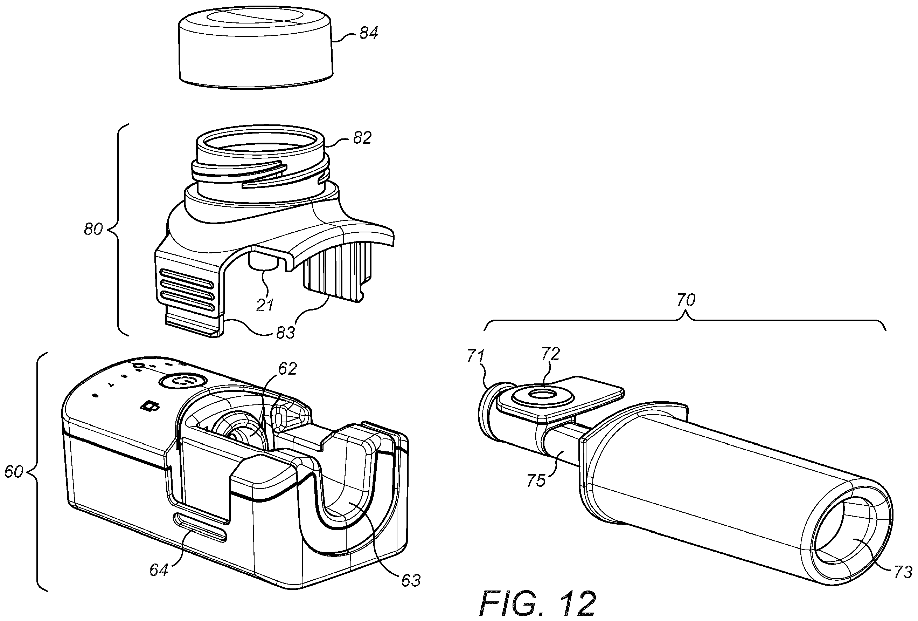

Figure 12 shows an expanded view of a vibrating membrane nebulizer device which is described in detail in EP2724741 and WO2013/098334, and which uses an aerosol generator of the type described in US 2010/0044460. The device comprises three parts: a base unit **60,** a mouthpiece component **70,** and an aerosol head **80.** The base unit **60** has one or more air inlet opening(s), an air outlet opening **62,** a groove **63** for receiving the mouthpiece component **70,** and one or more key lock members **64.** The base unit contains an electronic controller which controls the operation of the nebulizer. The mouthpiece component **70** has an air inlet opening **71** which is attachable to the air outlet opening **62** of the base unit **60,** a lateral opening **72** for receiving the aerosol generator **21,** and an aerosol outlet opening **73.** A mixing channel **75** extends from the air inlet opening **71** to the aerosol outlet opening **73.** The mouthpiece **70** is insertable into the groove **63** of the base unit **60.** The aerosol head **80** has the aerosol generator **21,** a filling chamber **82** for the liquid drug formulation to be nebulized, which is in fluid contact with the upper end of the aerosol generator **21,** and one or more key lock members **83** complementary to the key lock members **64** of the base unit **60.** A lid **84** closes the upper end of the filling chamber **82** and prevents contamination or spillage of the liquid during use. The base unit **60,** the mouthpiece **70** and the aerosol head **80** are detachably connectible with one another. The device is assembled by inserting the mouthpiece component **70** into the groove **63** in the base unit **60,** then placing the aerosol head **80** over the mouthpiece component **70** and engaging the key lock member(s) **83** of the aerosol head **80** with the complementary member(s) **64** of the base unit **60** by gentle pressure on both the aerosol head and the base unit. The aerosol generator **21** is positioned in the aerosol head **80** in such a way that when engaging the key lock member(s), the aerosol generator **21** is inserted into the lateral opening **72** of the mouthpiece **70.** This creates airtight connections between the aerosol generator **21** and the lateral opening **72** in the mouthpiece as well as between the air outlet opening **62** of the base unit **60** and the air inlet opening **71** of the mouthpiece **70.** The base unit **60,** the mouthpiece **70** and the aerosol head **80** can be separated by reversing these steps.

### Example

Aerosol generators as shown in Figure 3 were assembled using the piezo of Figures 4A & 4B. These were tested with saline solution, and were found to produce good aerosol output rates, similar to those produced by the aerosol generator shown in Figure 1. Thus the aerosol generator of the invention produces comparable performance to the known aerosol generator, but has fewer components and is simpler to manufacture.

## Claims

1. An aerosol generator (21) for a nebulizer, comprising:
• a vibratable membrane (25),
• a support member (22),
• an annular piezoelectric element (30) having a first surface (31), a second surface (32), an inner edge (36) and an outer edge (37),
• first (33) and second (34) conductive regions on the first (31) and second (32) surfaces respectively, wherein the second conductive region (34) extends across at least part of the inner edge (36) or the outer edge (37) onto the first surface (31) of the piezoelectric element to form a contact region (38), and
• a flexible connector (40) having a surface (42) which is an electrical insulator with first (47) and second (48) conductive regions which correspond to the first conductive region (34) and to the contact region (38) on the piezoelectric element respectively, and two legs (43, 44) for making electrical connection to a controller that provides a driving current to the piezoelectric element,
**characterized in that** the two legs (43, 44) are 'S'-shaped legs (43, 44).

2. An aerosol generator according to claim 1, wherein the second conductive region (34) extends across part of the outer edge (37a).

3. An aerosol generator according to claim 1, wherein the second conductive region (134) extends across part of the inner edge (136a).

4. An aerosol generator according to claim 1, wherein the second conductive region (234) extends across the whole of the outer edge (237).

5. An aerosol generator according to claim 1, wherein the second conductive region (334) extends across the whole of the inner edge (336).

6. An aerosol generator according to any of claims 1 to 5 wherein the first (33) and second (34) conductive regions cover most of the first (31) and the second (32) surfaces respectively.

7. An aerosol generator according to any of claims 1 to 6 wherein the legs lie in the plane of the flexible connector.

8. An aerosol generator according to any of claims 1 to 6, wherein the legs are arranged out of the plane of the flexible connector.

9. An aerosol generator according to any of claims 1 to 8 wherein the piezoelectric element is connected to the flexible connector (40) by a layer of anisotropic conducting paste (50) or anisotropic conductive adhesive transfer tape.

10. An aerosol generator according to any of claims 1 to 9 wherein the conductive regions (33, 34) on the piezoelectric element (30) are stencilled silver layers.

11. An aerosol generator according to any of claims 1 to 10, wherein the support member comprises a hollow tubular body having a flange at or close to a first end onto which the piezoelectric element is attached, and a second end into or onto which the membrane is mounted.

12. An aerosol generator according to any of claims 1 to 10 wherein the support member comprises an essentially planar annulus or disk, and wherein the membrane is in contact with the piezoelectric element, or the membrane and the piezoelectric element are mounted on the support member.

13. An inhalation device comprising an aerosol generator (21) according to any of claims 1 to 12.

14. An inhalation device according to claim 13 wherein the aerosol generator is an aerosol generator according to claim 11.

15. An inhalation device according to claim 14 comprising an aerosol head (80) comprising the aerosol generator (21); a base unit (60) having one or more an air inlet openings, an air outlet opening (62), and a groove (63); and a mouthpiece component (70) which is insertable into the groove (63) and which has an air inlet opening (71) that is attachable to the air outlet opening (62) of the base unit (60), a lateral opening (72) for receiving the aerosol generator (21), and an aerosol outlet opening (73); wherein the base unit (60), the mouthpiece component (70) and the aerosol head (80) are detachably connectible with each other.

## Patentansprüche

1. Aerosolgenerator (21) für einen Vernebler, umfassend:
• eine schwingfähige Membran (25),
• ein Stützelement (22),
• ein ringförmiges piezoelektrisches Element (30) mit einer ersten Oberfläche (31), einer zweiten Oberfläche (32), einem inneren Rand (36) und einem äußeren Rand (37),
• erste (33) und zweite (34) leitfähige Bereiche auf den ersten (31) bzw. zweiten (32) Oberflächen, wobei der zweite leitfähige Bereich (34) sich über mindestens einen Teil des inneren Rands (36) oder des äußeren Rands (37) auf die erste Oberfläche (31) des piezoelektrischen Elements erstreckt, um einen Kontaktbereich (38) zu bilden, und
• einen flexiblen Verbinder (40) mit einer Oberfläche (42), die ein elektrischer Isolator mit ersten (47) und zweiten (48) leitfähigen Bereichen ist, die dem ersten leitfähigen Bereich (34) bzw. dem Kontaktbereich (38) auf dem piezoelektrischen Element entsprechen, und mit zwei Schenkeln (43, 44) zur Herstellung einer elektrischen Verbindung mit einem Controller, der einen Antriebsstrom für das piezoelektrische Element bereitstellt,
**dadurch gekennzeichnet, dass** die zwei Schenkel (43, 44) "S"-förmige Schenkel (43, 44) sind.

2. Aerosolgenerator nach Anspruch 1, wobei der zweite leitfähige Bereich (34) sich über einen Teil des äußeren Rands (37a) erstreckt.

3. Aerosolgenerator nach Anspruch 1, wobei der zweite leitfähige Bereich (134) sich über einen Teil des inneren Rands (136a) erstreckt.

4. Aerosolgenerator nach Anspruch 1, wobei der zweite leitfähige Bereich (234) sich über den gesamten äußeren Rand (237) erstreckt.

5. Aerosolgenerator nach Anspruch 1, wobei der zweite leitfähige Bereich (334) sich über den gesamten inneren Rand (336) erstreckt.

6. Aerosolgenerator nach einem der Ansprüche 1 bis 5, wobei die ersten (33) und zweiten (34) leitfähigen Bereiche den größten Teil der ersten (31) bzw. zweiten (32) Oberflächen abdecken.

7. Aerosolgenerator nach einem der Ansprüche 1 bis 6, wobei die Schenkel in der Ebene des flexiblen Verbinders liegen.

8. Aerosolgenerator nach einem der Ansprüche 1 bis 6, wobei die Schenkel außerhalb der Ebene des flexiblen Verbinders angeordnet sind.

9. Aerosolgenerator nach einem der Ansprüche 1 bis 8, wobei das piezoelektrische Element mittels einer Schicht aus einer anisotropen leitenden Paste (50) oder eines anisotropen leitfähigen Transferklebebands mit dem flexiblen Verbinder (40) verbunden ist.

10. Aerosolgenerator nach einem der Ansprüche 1 bis 9, wobei die leitfähigen Bereiche (33, 34) auf dem piezoelektrischen Element (30) schablonierte Silberschichten sind.

11. Aerosolgenerator nach einem der Ansprüche 1 bis 10, wobei das Stützelement einen hohlen röhrenförmigen Körper mit einem Flansch an oder nahe bei einem ersten Ende, an dem das piezoelektrische Element befestigt ist, und einem zweiten Ende, in oder auf dem die Membran befestigt ist, umfasst.

12. Aerosolgenerator nach einem der Ansprüche 1 bis 10, wobei das Stützelement eine/n im Wesentlichen ebene/n Kreisring oder Scheibe umfasst und wobei die Membran mit dem piezoelektrischen Element in Kontakt ist oder die Membran und das piezoelektrische Element auf dem Stützelement befestigt sind.

13. Inhalationsvorrichtung, umfassend einen Aerosolgenerator (21) nach einem der Ansprüche 1 bis 12.

14. Inhalationsvorrichtung nach Anspruch 13, wobei der Aerosolgenerator ein Aerosolgenerator nach Anspruch 11 ist.

15. Inhalationsvorrichtung nach Anspruch 14, umfassend einen Aerosolkopf (80), der den Aerosolgenerator (21) umfasst; eine Basiseinheit (60) mit ein oder mehr Lufteinlassöffnungen, einer Luftauslassöffnung (62) und einer Rille (63); und eine Mundstückkomponente (70), die in die Rille (63) einführbar ist und eine Lufteinlassöffnung (71), die mit der Luftauslassöffnung (62) der Basiseinheit (60) verbindbar ist, eine seitliche Öffnung (72) zur Aufnahme des Aerosolgenerators (21) und eine Aerosolauslassöffnung (73) aufweist; wobei die Basiseinheit (60), die Mundstückkomponente (70) und der Aerosolkopf (80) lösbar miteinander verbindbar sind.

## Revendications

1. Générateur d'aérosol (21) pour un nébuliseur, comprenant :
• une membrane vibrante (25),
• un élément de support (22),
• un élément piézoélectrique annulaire (30) ayant une première surface (31), une seconde surface (32), un bord intérieur (36) et un bord extérieur (37),
• des première (33) et seconde (34) régions conductrices sur les première (31) et seconde (32) surfaces respectivement, la seconde région conductrice (34) s'étendant sur au moins une partie du bord intérieur (36) ou du bord extérieur (37) sur la première surface (31) de l'élément piézoélectrique pour former une région de contact (38), et
• un connecteur flexible (40) ayant une surface (42) qui est un isolant électrique avec des première (47) et seconde (48) régions conductrices qui correspondent à la première région conductrice (34) et à la région de contact (38) sur l'élément piézoélectrique respectivement, et deux pattes (43, 44) pour établir une connexion électrique à un dispositif de commande qui fournit un courant d'entraînement à l'élément piézoélectrique,
**caractérisé en ce que** les deux pattes (43, 44) sont des pattes en forme de 'S' (43, 44).

2. Générateur d'aérosol selon la revendication 1, la seconde région conductrice (34) s'étendant sur une partie du bord extérieur (37a).

3. Générateur d'aérosol selon la revendication 1, la seconde région conductrice (134) s'étendant sur une partie du bord intérieur (136a).

4. Générateur d'aérosol selon la revendication 1, la seconde région conductrice (234) s'étendant sur l'ensemble du bord extérieur (237).

5. Générateur d'aérosol selon la revendication 1, la seconde région conductrice (334) s'étendant sur l'ensemble du bord intérieur (336).

6. Générateur d'aérosol selon l'une quelconque des revendications 1 à 5, les première (33) et seconde (34) régions conductrices couvrant la majeure partie de la première (31) et de la seconde (32) surfaces respectivement.

7. Générateur d'aérosol selon l'une quelconque des revendications 1 à 6, les pattes se trouvant dans le plan du connecteur flexible.

8. Générateur d'aérosol selon l'une quelconque des revendications 1 à 6, les pattes étant disposées hors du plan du connecteur flexible.

9. Générateur d'aérosol selon l'une quelconque des revendications 1 à 8, l'élément piézoélectrique étant relié au connecteur flexible (40) par une couche de pâte conductrice anisotrope (50) ou une bande de transfert adhésive conductrice anisotrope.

10. Générateur d'aérosol selon l'une quelconque des revendications 1 à 9, les régions conductrices (33, 34) sur l'élément piézoélectrique (30) étant des couches d'argent au pochoir.

11. Générateur d'aérosol selon l'une quelconque des revendications 1 à 10, l'élément de support comprenant un corps tubulaire creux ayant une bride à ou près d'une première extrémité sur laquelle l'élément piézoélectrique est fixé, et une seconde extrémité dans ou sur laquelle la membrane est montée.

12. Générateur d'aérosol selon l'une quelconque des revendications 1 à 10, l'élément de support comprenant un anneau ou un disque essentiellement plan, et la membrane étant en contact avec l'élément piézoélectrique, ou la membrane et l'élément piézoélectrique étant montés sur l'élément de support.

13. Dispositif d'inhalation comprenant un générateur d'aérosol (21) selon l'une quelconque des revendications 1 à 12.

14. Dispositif d'inhalation selon la revendication 13, le générateur d'aérosol étant un générateur d'aérosol selon la revendication 11.

15. Dispositif d'inhalation selon la revendication 14, comprenant une tête d'aérosol (80) comprenant le générateur d'aérosol (21) ; une unité de base (60) présentant une ou plusieurs ouvertures d'entrée d'air, une ouverture de sortie d'air (62), et une rainure (63) ; et un embout buccal (70) qui peut être inséré dans la rainure (63) et qui possède une ouverture d'entrée d'air (71) qui peut être fixée à l'ouverture de sortie d'air (62) de l'unité de base (60), une ouverture latérale (72) pour recevoir le générateur d'aérosol (21) et une ouverture de sortie d'aérosol (73) ; l'unité de base (60), l'embout buccal (70) et la tête d'aérosol (80) étant connectables de manière amovible les uns aux autres.
